# EUROPÄISCHE PATENTSCHRIFT

(11) **EP 0 399 545 B1**
(45) Veröffentlichungstag und Bekanntmachung des Hinweises auf die Patenterteilung: **12.04.1995**
(21) Anmeldenummer: 90109939.0
(22) Anmeldetag: 25.05.1990
(51) Int. Cl.: C12N 5/00

(54) **Verfahren zur Dekontamination mycoplasmenhaltiger Zellkulturen**
Process for decontaminating cell cultures infested by mycoplasms
Procédé pour la décontamination de cultures de cellules infestées par des mycoplasmes

(30) Priorität: 26.05.1989 DE 3917163
(43) Veröffentlichungstag der Anmeldung: 28.11.1990
(73) Patentinhaber: BEHRINGWERKE Aktiengesellschaft, 35001 Marburg (DE)
(72) Erfinder: Langner, Klaus-Dieter, Dr., D-3550 Marburg (DE)

(56) Entgegenhaltungen:
- EP-A- 0 221 493
- EP-A- 0 348 947
- US-A- 4 387 161
- EXPERIMENTAL CELL RESEARCH, Band 139, 1982, Seiten 199-205, New York, US; G:J: MCGARRITY et al.

## Beschreibung

Die Erfindung betrifft ein Verfahren zur Dekontamination mycoplasmenhaltiger Zellkulturen. Mit Mycoplasmen infizierte Zellkulturen werden dabei in einem ersten Schritt einer Antibiotikabehandlung unterzogen, wobei man anschließend in einem zweiten Schritt noch infizierte Zellen durch 6-Methylpurin Desoxyribose-Lösung eliminiert.

Mycoplasmen sind bakterienähnliche Mikroorganismen, die weder eine Zellwand noch eine Schleimkapsel synthetisieren. Sie vermehren sich sowohl in Zellkulturen (cytoadsorbiert) als auch in zellfreiem Medium. Charakteristisch für Mycoplasmen ist der Bedarf von Cholesterin oder anderen Steroidverbindungen.

Die Kontamination von Zellkulturen durch Mycoplasmen ist ein weit verbreitetes Problem in der Zelltechnologie. Sie wurde zuerst von Robinson (L.B. Robinson et al., (1956), Science, 124, 1147) dokumentiert. Mittlerweile findet man im Labormaßstab bei permanent wachsenden Zellkulturen eine durchschnittliche Kontaminationsrate von ca. 15%. Bei der großtechnischen Kultivierung können sogar bis 90% aller Kulturen von Mycoplasmen befallen sein. Dies stellt für die biotechnologische Produktion von rekombinanten Proteinen ein großes Problem dar, da pharmazeutische Produkte, welche in Animalzellen synthetisiert werden, absolut frei von Mycoplasmenantigenen sein müssen.

Die größten Infektionsquellen stellen kontaminierte Seren und bereits infizierte Zellkulturen dar. Die Übertragung erfolgt meist über Tröpfcheninfektion oder Aerosole. Eine infizierte Kultur enthält zwischen 10⁶-10⁹ colony forming units (CFU) pro Milliliter konditioniertes Medium, ohne daß eine Trübung des Mediums oder ein charakteristischer Geruch festzustellen wäre.

Die Anwesenheit bestimmter Mycoplasmenstämme führt, bedingt durch extensive Säureproduktion, zu degenerativen Effekten in der Zellkultur. Die am häufigsten vorkommenden Mycoplasmenstämme wie M. arginini, M. orale, M. hyorhinis und A. laidlawii proliferieren, ohne daß morphologische Veränderungen der kontaminierten Zellinie beobachtet werden. Jedoch findet man in Mycoplasmen kontaminierten Kulturen häufig Chromosomen-Aberrationen und einen veränderten Zellmetabolismus.

In der Praxis gibt es mittlerweile eine Reihe von Testsystemen, mit deren Hilfe eine Mycoplasmen-Kontamination nachgewiesen werden kann. Ein einfacher und sensitiver Nachweis, auch von nicht cytoadsorbierten Mycoplasmen, beruht auf der durch Mycoplasmen vermittelten Cytotoxizität von 6-Methylpurin Derivaten.

Diese Cytotoxizität liegt dem Enzym Adenosin Phosphorylase zugrunde, welches in Mycoplasmen, jedoch nicht in Animalzellen synthetisiert wird. In Anwesenheit dieses Enzyms werden 6-Methylpurin Desoxyribose (6-MPDR) oder 6-Methylpurin Ribose (6-MPR) gespalten, wobei 6-Methylpurin (6-MP) als die für die Zellen toxische Substanz frei wird (G.J. McGarrity, et al. (1982), Exp. Cell Research, 139, 199). Die Folge ist eine Lyse der mit Mycoplasmen kontaminierten Zellkultur, da bereits Konzentrationen von kleiner 5 uM des Spaltproduktes 6-MP für die Zellen toxisch sind. Da alle relevanten Mycoplasmenstämme Adenosin Phosphorylase enthalten, werden mit diesem Testsystem auch alle möglichen Kontaminationsquellen erfaßt.

Die Dekontamination von mycoplasmenhaltigen Kulturen gilt bislang als schwierig und aufwendig. Eine Möglichkeit ist die Passage der kontaminierten Kultur in nackten Mäusen. Dieses Verfahren wird bei Hybridomakulturen angewendet, erfordert jedoch einen großen technischen Aufwand. Bei anderen Zellspezies (z.B. BHK-Zellen) besteht außerdem die Gefahr, daß die Zellen bei dieser Prozedur verlorengehen können. Eine weitere Möglichkeit stellt die Behandlung mit verschiedenen Antibiotika dar. Es gibt eine Reihe von Substanzen, die für Animalzellen relativ gut verträglich sind und mit denen die Mycoplasmen zumindest kurzzeitig entfernt werden können. Oftmals werden die verschiedenen Mycoplasmenstämme in ihrem Wachstum jedoch nur repremiert, so daß nach einigen Passagen die Kultur wieder kontaminiert ist. Außerdem besteht bei mehrmaliger Anwendung die Gefahr, daß sich Resistenzen gegen die verschiedenen Antibiotika ausbilden.

Die Erfindung betrifft folglich ein Verfahren zur Dekontamination von mycoplasmenhaltigen Zellkulturen, wobei in einem ersten Schritt gegen Mycoplasmen wirksame Antibiotika eingesetzt werden. In einem weiteren Schritt werden anschließend noch mycoplasmenhaltige Zellen bzw. Zellklone durch Kultivierung in Anwesenheit von 6-Methylpurin Desoxyribose eliminiert, so daß nur die tatsächlich mycoplasmenfreien Zellen oder Zellklone übrigbleiben d.h. überleben. Vorzugsweise werden als Antibiotika Thiamulin und Minocyclin eingesetzt und 6-Methylpurin Desoxyribose in einer Konzentration von 50 »M verwendet.

Die Erfindung betrifft ferner die auf solche Weise gewonnenen mycoplasmenfreien Zellkulturen sowie deren Verwendung, insbesondere bei der gentechnischen Herstellung von Fremdproteinen in diesen Zellen. Die Erfindung ist zusätzlich in den Patentansprüchen und dem Beispiel offenbart.

### Beispiel: Dekontamination mycoplasmenhaltiger Hamsterzellen

Nagerzellen stellen ein für die Expression heterologer rekombinanter Proteine bevorzugtes Expressionssystem dar. Am häufigsten werden CHO-(chinese hamster ovary) und BHK-(baby hamster kidney) Zellen benutzt. Beide Zellinien sind etablierte Kulturen und werden bereits für die großtechnische Produktion rekombinanter Proteine verwendet.

Über ein aufwendiges Transfektions-, Selektions- und Klonierungsverfahren wurden BHK- und CHO-Klone isoliert, die ein bestimmtes Protein (z.B. Faktor VIII:C oder AT III) sezernierten. Bei einigen dieser Klone wurde eine Mycoplasmen-Kontamination nachgewiesen.

Um nicht noch einmal das zeitaufwendige Transfektions-und Selektionsverfahren mit mycoplasmenfreien Zellen zu wiederholen, wurde das folgende Verfahren zur Dekontamination angewandt: Die kontaminierten Zellkulturen wurden zunächst alternierend je 2 Passagen mit den Antibiotika Thiamulin und Minocyclin (Sebio, Konzentration nach Herstellerangaben) behandelt. Anschließend wurden die Zellen 4 Passagen völlig ohne Antibiotika gehalten. Nach Überprufung im Mycoplasmentest wurden die mycoplasmenfreien Kulturen klonvereinzelt. Hierzu wurden die Zellen über "limiting dilution" in einer Zelldichte von 2 Zellen/Vertiefung und 10 Zellen/Vertiefung auf einer 96 Loch-Schale ausgesät. Das Anlegen von jeweils 5 Vertiefungen pro Verdünnung je Kultur hatte sich dabei als ausreichend erwiesen. Nach einer Anwachsphase von 1-2 Tagen wurde den Kulturen jeweils 5 »l/Vertiefung einer 1 mM 6-MethylpurinDesoxyribose Lösung (6-MPDR) zugesetzt (Endkonzentration 50 »M/Vertiefung). Anschließend wurden die Zellen 5-6 Tage (bis zum Erreichen der Konfluenz) mit dieser Lösung inkubiert.

Zellen, die noch schwach mit cytoadsorbierten Mycoplasmen kontaminiert waren, starben unter diesen Bedingungen ab. Anschließend wurden die Zellen auf eine 24-Lochplatte und später auf eine 6-Lochplatte überführt und ohne 6-MPDR gehalten.

Nach dieser Behandlung wurden erneut verschiedene Mycoplasmentests durchgeführt (mikrobiologische Kultivierung und Mycoplasmen vermittelte Cytotoxizität). Nach negativem Befund wurden die Klone teils eingefroren und teils weiter in Kultur gehalten. Nach jeweils 5, 10, 15 und 20 Passagen wurden die Zellen erneut auf Mycoplasmen-Kontamination hin untersucht, wobei bei allen Kulturen, die nach dem oben beschriebenen Verfahren behandelt wurden, immer ein negativer Befund festgestellt werden konnte.

Parallel hierzu wurden die Zellen nur mit den oben beschriebenen Antibiotika behandelt, klonvereinzelt und nicht in Anwesenheit von 6-MPDR gehalten. Anschließend wurden die Zellen eingefroren und teilweise zur Kontrolle in Kultur gehalten. Nach 5, 10, 15 und 20 passagen wurden ebenfalls Mycoplasmentests durchgeführt, wobei bei ca. 30% der Kulturen nach Passage 10 wiederum Mycoplasmen nachweisbar waren. Dabei wurden die Zellen bei Bedingungen gehalten, unter denen eine Neuinfektion mit Mycoplasmen auszuschließen war.

Dieses Verfahren wurde auch bei menschlichen Zellkulturen (Hela- und KB-Zellen) sowie bei Affenzellen (COS-Zellen) mit dem gleichen Erfolg angewendet.

## Patentansprüche

1. Verfahren zur Dekontamination Von mycoplasmenhaltigen Zellkulturen, umfassend die Schritte
a) Behandlung mit für Mycoplasmen wirksamen Antibiotika,
b) anschließende Vereinzelung der behandelten Zellen
c) Behandlung der vereinzelten Zellen mit 6-Methylpurin-Deoxyribose

2. Verfahren nach Anspruch 1, dadurch gekennzeichnet, daß als Antibiotika Thiamulin und Minocyclin verwendet werden.

3. Verfahren nach Anspruch 1 oder Anspruch 2, dadurch gekennzeichnet, daß 6-Methylpurin-Desoxyribose in einer Endkonzentration von 50 »M eingesetzt wird.

4. Verwendung eines Verfahrens nach Anspruch 1, 2 oder 3 zur Herstellung von mycoplasmenfreien Zellkulturen.

5. Verfahren nach Anspruch 1, 2 oder 3, dadurch gekennzeichnet, daß die dekontaminierten Zellinien vom Menschen oder Tier stammen.

## Claims

1. A process for the decontamination of cell cultures containing mycoplasmas, which comprises the steps
a) treatment with antibiotics effective for mycoplasmas,
b) subsequent singularization of the treated cells,
c) treatment of the singularized cells with 6-methylpurine deoxyribose.

2. The process as claimed in claim 1, wherein the antibiotics used are tiamulin and minocycline.

3. The process as claimed in claim 1 or claim 2, wherein 6-methylpurine deoxyribose is used in a final concentration of 50 »M.

4. The use of a process as claimed in claim 1, 2 or 3 for the production of mycoplasma-free cell cultures.

5. The process as claimed in claim 1, 2 or 3, wherein the decontaminated cell lines derive from humans or animals.

## Revendications

1. Procédé pour la décontamination de cultures de cellules contenant des mycoplasmes, comprenant les étapes suivantes:
a) traitement par des antibiotiques actifs pour des mycoplasmes,
b) isolement subséquent des cellules traitées,
c) traitement des cellules isolées par du 6-méthylpurine-désoxyribose.

2. Procédé selon la revendication 1, caractérisé en ce que, en tant qu'antibiotiques, on utilise la thiamuline et la minocycline.

3. Procédé selon la revendication 1 ou 2, caractérisé en ce que le 6-méthylpurine-désoxyribose est utilisé à une concentration finale de 50 »M.

4. Utilisation d'un procédé selon la revendication 1, 2 ou 3, pour la production de cultures de cellules exemptes de mycoplasmes.

5. Procédé selon la revendication 1, 2 ou 3, caractérisé en ce que les lignées cellulaires décontaminées sont d'origine humaine ou animale.
